(19) Europäisches Patentamt
European Patent Office
Office européen des brevets

(11) **EP 3 487 412 B1**

(12) **FASCICULE DE BREVET EUROPEEN**

(45) Date de publication et mention
de la délivrance du brevet:
**14.10.2020 Bulletin 2020/42**

(51) Int Cl.:
*A61B 8/08* (2006.01)     *A61B 8/00* (2006.01)
*G01S 7/52* (2006.01)

(21) Numéro de dépôt: **17748439.1**

(22) Date de dépôt: **24.07.2017**

(86) Numéro de dépôt international:
**PCT/EP2017/068687**

(87) Numéro de publication internationale:
**WO 2018/019791 (01.02.2018 Gazette 2018/05)**

(54) **PROCEDE DE MESURE D'UN PARAMETRE VISCOELASTIQUE D'UN ORGANE HUMAIN OU ANIMAL**

VERFAHREN ZUR MESSUNG EINES VISKOELASTISCHEN PARAMETERS EINES MENSCHLICHEN ODER TIERISCHEN ORGANS

METHOD FOR MEASURING A VISCOELASTIC PARAMETER OF A HUMAN OR ANIMAL ORGAN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.07.2016 FR 1657126**

(43) Date de publication de la demande:
**29.05.2019 Bulletin 2019/22**

(73) Titulaire: **Echosens**
**75013 Paris (FR)**

(72) Inventeurs:
• **AUDIERE, Stéphane**
  **75012 Paris (FR)**
• **SANDRIN, Laurent**
  **92340 Bourg-la-Reine (FR)**

(74) Mandataire: **Cabinet Camus Lebkiri**
**25 rue de Maubeuge**
**75009 Paris (FR)**

(56) Documents cités:
**US-A1- 2013 024 136     US-A1- 2015 374 338**

• **MAGALI SASSO ET AL: "Liver Steatosis Assessed by Controlled Attenuation Parameter (CAP) Measured with the XL Probe of the FibroScan: A Pilot Study Assessing Diagnostic Accuracy", ULTRASOUND IN MEDICINE AND BIOLOGY., vol. 42, no. 1, 2 janvier 2016 (2016-01-02), pages 92-103, XP055365061, US ISSN: 0301-5629, DOI: 10.1016/j.ultrasmedbio.2015.08.008**
• **NIGHTINGALE KATHRYN ET AL: "Derivation and analysis of viscoelastic properties in human liver: impact of frequency on fibrosis and steatosis staging", IEEE TRANSACTIONS ON ULTRASONICS, FERROELECTRICS AND FREQUENCY CONTROL, IEEE, US, vol. 62, no. 1, 2 janvier 2015 (2015-01-02), pages 165-175, XP011569772, ISSN: 0885-3010, DOI: 10.1109/TUFFC.2014.006653 [extrait le 2015-01-06]**

## Description

## DOMAINE DE L'INVENTION

La présente invention concerne le domaine de la mesure des propriétés viscoélastiques d'un tissu biologique. Plus spécifiquement, l'invention porte sur un procédé de mesure d'un paramètre viscoélastique à l'aide d'impulsions ultrasonores multifréquences.

## ETAT DE L'ART

Plusieurs techniques non-invasives pour la mesure des propriétés viscoélastiques des organes humains ou animaux ont été développées.

Parmi ces techniques l'élastographie impulsionnelle est particulièrement efficace dans le suivi des affections des tissus mous et notamment du foie humain. Par exemple, la demanderesse a développé et commercialisé un dispositif, Fibroscan™, qui est désormais une référence pour le diagnostic et le suivi de la fibrose hépatique. Ce dispositif est notamment illustré dans les brevets EP 1 169 636 et EP 1 531 733 déposés par la demanderesse. Cette affection peut être la conséquence de maladies comme les hépatites ou le cancer du foie et elle se caractérise par une perte d'élasticité du foie. La technologie développée par le déposant et décrite dans les brevets cités est connue avec le nom « Vibration Controlled Transient Elastography » (ou VCTE).

La technologie VCTE porte sur le suivi de la propagation d'une onde de cisaillement à l'intérieur du tissu à caractériser. L'onde de cisaillement est générée à l'aide d'une vibration mécanique et le suivi de sa propagation est réalisé à l'aide d'ultrasons. Le suivi de la propagation de l'onde de cisaillement permet de mesurer sa vitesse de propagation, qui dépend directement de l'élasticité du milieu de propagation.

D'autres affections du foie sont aujourd'hui plus difficiles à évaluer. C'est le cas par exemple de la stéatose hépatique, qui se manifeste par une accumulation de lipides à niveau des cellules du foie appelées hépatocytes. Dans certains cas il s'agit d'une situation bénigne et réversible. Néanmoins, elle peut être associée à une inflammation chronique (stéato-hépatites), notamment quand elle est accompagnée par une assomption d'alcool élevée, par l'obésité ou bien par des maladies métaboliques. Dans les maladies chroniques du foie la stéatose peut être un cofacteur dans la progression de la fibrose.

La stéatose du foie peut être évaluée en mesurant l'absorption des ultrasons qui se propagent à l'intérieur du foie (voir par exemple M. Sasso et al « Controlled attenuation parameter (cap): a novel vcte guided ultrasonic attenuation measurement for the evaluation of hepatic steatosis: preliminary study and validation in a cohort of patients with chronic liver disease from various causes » dans Ultrasound in Medicicine and Biology Vol. 36, Numéro 11, 2010, et M. Sasso et al. « Liver steatosis assessed by controlled attenuation parameter (cap) measured with the xl probe of the fibroscan: a pilot study assessing diagnostic accuracy» dans Ultrasound in Medicine and Biology, Volume 42, Numéro 1, 2015).

Il est important donc de pouvoir mesurer la quantité de stéatose présente dans le foie et si possible d'associer à la mesure de stéatose une évaluation de l'élasticité du foie. Préférentiellement ces diagnostics doivent pouvoir être réalisés au cours de la même consultation médicale et avec des techniques non-invasives.

Avantageusement ces mesures d'atténuation sont couplées à la mesure d'une autre propriété viscoélastique du foie comme par exemple son élasticité. Cette technique est décrite par la demande de brevet EP2477551 déposée par la demanderesse.

La publication US2015/0374338 décrit aussi l'élastographie ultrasonore.

Néanmoins, les solutions techniques connues fournissent des mesures avec un rapport signal sur bruit qui peut être très faible notamment dans le cas de patients obèses - dont le foie présente souvent de la stéatose - à cause de l'atténuation élevée des impulsions ultrasonores dans les tissus sous cutanés (graisse notamment), ainsi que dans le milieu à mesurer lui-même.

## RESUME DE L'INVENTION

Dans ce contexte, la présente invention vise à fournir un procédé de mesure d'au moins une propriété d'un organe humain ou animal ou d'un tissu viscoélastique permettant de réaliser rapidement des mesures fiables, reproductibles et ayant un plus grand rapport signal sur bruit dans une large cohorte de patients comprenant aussi les patients obèses.

A cette fin, l'invention propose notamment un procédé de mesure d'au moins un paramètre viscoélastique d'un organe humain ou animal, ledit procédé comportant les étapes suivantes :

- émission par un transducteur ultrasonore d'une pluralité de tirs ultrasonores, lesdits tirs ultrasonores se propageant dans l'organe à caractériser ;
- réception par le transducteur ultrasonore et enregistrement des signaux ultrasonores réfléchis ;
- détermination d'au moins un paramètre viscoélastique de l'organe à partir des signaux ultrasonores enregistrés.

Dans le procédé selon l'invention :

- la pluralité de tirs ultrasonores est formée par la répétition de K groupes de tirs, lesdits groupes étant séparés temporellement, K étant supérieur ou égale à 1 ;
- chacun des K groupes de tirs ultrasonores est formé par la répétition avec une cadence PRF2 de $M_K$ blocs(s) de tirs ultrasonores, $M_K$ étant supérieur ou égale à 1 ;

- chacun desdits $M_K$ blocs est composé par N tirs ultrasonores, N étant supérieur ou égale à 1, PRF1 étant la cadence d'émission des N tirs quand N est choisi supérieur à 1 ;
- les N tirs ultrasonores sont repartis sur P fréquences, P étant compris entre 1 et N, au moins deux tirs ultrasonores appartenant à deux blocs différents ayant fréquences différentes

On entend par paramètre viscoélastique d'un organe humain ou animal un paramètre comme par exemple l'atténuation des signaux ultrasonores. Dans le cas du foie humain ce paramètre est lié à la stéatose, à savoir la concentration de lipides dans les tissus.

On entend par organe humain ou animal un tissu biologique comme par exemple le foie. Dans ce qui suit on appellera ce tissu biologique un organe humain ou animal ou indifféremment un tissu ou un milieu viscoélastique.

On entend par transducteur ultrasonore un dispositif disposé à proximité du tissu à étudier et capable à la fois d'émettre des ultrasons qui se propagent à l'intérieur du tissu et de détecter les signaux ultrasonores réfléchis. Le transducteur peut être également formé par une multiplicité d'éléments destinés à former une barrette.

Avantageusement, dans le cas du foie humain, l'invention permet d'obtenir une meilleure évaluation de la stéatose en augmentant la qualité, la reproductibilité et la rapidité des mesures.

Selon un mode de réalisation, l'enregistrement des signaux ultrasonores réfléchis peut être effectué à chaque tir ultrasonore émis.

Le procédé selon l'invention prévoit l'émission d'une multiplicité de tirs ultrasonores repartie en K groupes, les K groupes étant temporellement séparés. Chacun des K groupes comprend une répétition de $M_K$ blocs de tirs ultrasonores avec une cadence PRF2. On entend par cadence PRF2 le taux de répétition de l'émission des M blocs de tirs ultrasonores. Par exemple une cadence PRF2 de 1 Hz comporte l'émission d'un bloc de tirs par seconde, une cadence PRF2 de 60Hz comporte l'émission de 60 blocs de tirs par seconde, c'est-à-dire un bloc tous les 1/60 de seconde.

Chacun des $M_K$ blocs émis est composé par N tirs. Les N tirs composant chaque bloc sont émis avec une cadence, c'est-à-dire un taux de répétition, PRF1.

Quand une impulsion ultrasonore se propage à l'intérieur du tissu à caractériser, son intensité en fonction de la distance parcourue z est donnée par la formule :

$$I(z) = I_0 e^{-\alpha(f)z}$$

Ici I(z) est l'intensité de l'impulsion en fonction de la distance parcourue z, $I_0$ est l'intensité émise et a(f) est le coefficient d'absorption du tissu, exprimé en dB m$^{-1}$. Le coefficient d'absorption dépend de plusieurs paramètres et notamment de la fréquence des signaux ultrasonores, f, et des propriétés du tissu. Plus particulièrement, dans le cas du foie a(f) dépend du contenu en lipides du tissu et constitue donc une mesure de la stéatose.

Les N tirs ultrasonores sont répartis sur P fréquences. Les P fréquences sont différentes quand P>1. Cela permet d'exploiter le fait que le transducteur ultrasonore utilisé pour la mise en œuvre du procédé peut émettre des impulsions ayant différentes fréquences centrales. L'ensemble des fréquences qu'un transducteur ultrasonore peut émettre est appelé la bande passante du transducteur. L'invention permet donc de mesurer des signaux ultrasonores réfléchis à plusieurs fréquences et donc de mesurer a(f) pour différentes valeurs de f.

Un avantage de l'invention est celui d'augmenter le signal mesuré car plusieurs impulsions à des fréquences différentes sont envoyées dans le tissu. Des mesures avec un rapport signal sur bruit plus élevé sont alors réalisées. De plus, cette augmentation du rapport signal sur bruit est réalisée sans augmenter l'intensité associée à chaque impulsion : la puissance totale envoyée est partagée par plusieurs impulsions étalées sur une large plage de fréquences. La plage de fréquences accessibles correspond à la bande passante du transducteur ultrasonore.

Dans le cas du foie humain, la fréquence de référence pour la mesure de la stéatose est de 3.5MHz (fréquence centrale). Dans une plage de quelques MHz autour de cette fréquence, le coefficient a(f) varie à peu près linéairement avec la fréquence f.

Grâce à l'invention il est possible de mesurer a(f) avec un meilleur rapport signal sur bruit pour plusieurs valeurs de f autour de la fréquence centrale de 3.5MHz et, avec par exemple une simple régression linéaire, d'obtenir une estimation plus précise de la valeur de référence a(f=3.5 MHz).

Outre les caractéristiques principales mentionnées plus haut, le procédé pour la mesure d'au moins une propriété de tissu biologique selon l'invention peut présenter une ou plusieurs caractéristiques supplémentaires ci-dessous, considérées individuellement ou selon toutes les combinaisons techniquement possibles :

- le paramètre viscoélastique mesuré est un paramètre d'atténuation ultrasonore ;
- la cadence PRF2 est supérieure ou égale à 1 Hz et la cadence PRF1 est supérieure ou égale à 1kHz ;
- les P fréquences différentes et les largeurs spectrales des N tirs ultrasonores sont choisies pour couvrir sensiblement la bande passante du transducteur ultrasonore ;
- P est égal à N ;
- M est compris entre 10 et 10000, P est compris entre 1 et 11, N est compris entre 2 et 11, PRF2 est compris entre 5 Hz et 500 Hz et PRF1 est compris entre 1 kHz et 10 kHz ;

- Fc est la fréquence centrale du transducteur ultrasonore, P est égal à 5 et les fréquences choisies sont 0.5*Fc < F1 < 0.7*Fc ; 0.7*Fc < F2 < 0.9*Fc ; 0.9*Fc < F3 < 1.1*Fc ; 1.1*Fc < F4 < 1.3*Fc ; 1.3*Fc < F5 < 1.5*Fc ;

La présente demande a également pour objet un dispositif pour la mise en œuvre du procédé, ledit dispositif comprenant :

⚬ un transducteur ultrasonore adapté à l'émission et à la réception en temps réel de signaux ultrasonores et adapté pour être disposé au contact d'une surface extérieure du milieu viscoélastique ;
⚬ des moyens de commande dudit transducteur ultrasonore pour l'émission d'une pluralité de tirs ultrasonores, ladite pluralité de tirs ultrasonores étant formée par K groupes de tirs, lesdits groupes étant séparés temporellement, K étant supérieur ou égal à 1, chacun des K groupes de tirs ultrasonores étant formé par la répétition avec une cadence PRF2 de $M_K$ groupe(s) de tirs ultrasonores, $M_K$ étant supérieur ou égale à 1 ; chacun desdits $M_K$ blocs étant composé par N tirs ultrasonores, N étant supérieur ou égale à 1, PRF1 étant la cadence d'émission des N tirs quand N est choisi supérieur à 1 ; les N tirs ultrasonores étant répartis sur P fréquences, P étant compris entre 1 et N, au moins deux tirs ultrasonores appartenant à deux blocs différents ayant fréquences différentes ;
⚬ des moyens d'enregistrement et de traitement des signaux ultrasonores réfléchis adaptés pour déterminer au moins un paramètre viscoélastique de l'organe.

La présente demande a également pour objet un deuxième procédé selon la revendication 8.

La présente invention a également pour objet un dispositif selon la revendication 10 pour la mise en œuvre du deuxième procédé.

## LISTE DES FIGURES

D'autres caractéristiques et avantages de l'invention ressortiront clairement de la description qui en est donnée ci-après, à titre indicatif et nullement limitatif, en référence aux figures annexées ci-jointes parmi lesquelles :

- la figure 1 montre les différentes étapes du premier procédé selon l'invention ;
- la figure 2 illustre une pluralité de tirs ultrasonores formée, à titre d'exemple, par K = 3 groupes, les trois groupes K=1, K=2 et K=3 étant formé respectivement par $M_{K=1}$, $M_{K=2}$ et $M_{K=3}$ blocs de tirs ;
- la figure 3 montre la structure d'un des K groupes de tirs ultrasonore de la figure 2 ; à titre d'exemple ce groupe est formé par $M_K$ = 5 blocs de tirs, lesdits blocs émis avec une cadence PRF2, chacun des $M_K$ blocs étant formé par N tirs ultrasonores, lesdits N tirs étant émis avec une cadence PRF1 ;
- la figure 4 montre les différentes étapes du deuxième procédé selon l'invention ;
- la figure 5 illustre schématiquement un dispositif pour la mise en œuvre du procédé de la figure 1 ;
- la figure 6 illustre schématiquement un dispositif pour la mise en œuvre du procédé de la figure 4 ;
- la figure 7 montre une représentation de la sonde du dispositif de la figure 6.

## DESCRIPTION DETAILLEE DE L'INVENTION

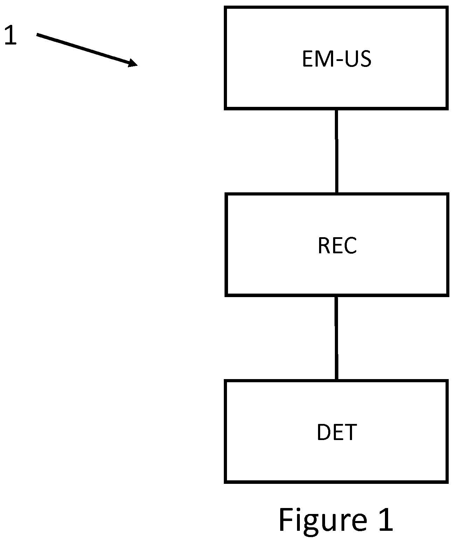

La figure 1 illustre les différentes étapes du premier procédé 1 selon l'invention. Le procédé 1 comporte ici trois étapes :

- une émission EM-US par un transducteur ultrasonore US d'une pluralité de tirs ultrasonores qui se propagent dans l'organe à caractériser,
- un enregistrement REC par le même transducteur ultrasonore US des signaux ultrasonores réfléchis,
- une détermination DET d'au moins un paramètre viscoélastique de l'organe à partir des signaux enregistrés.

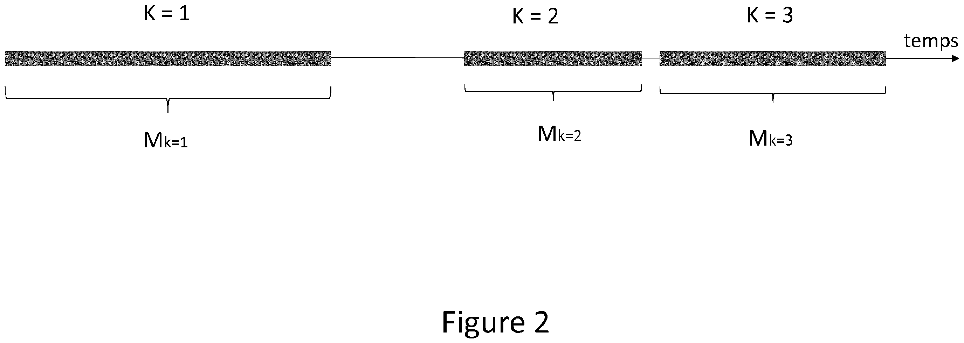

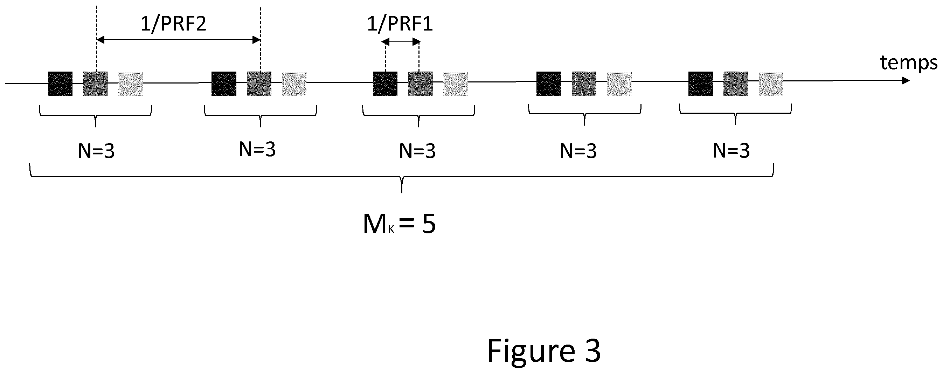

Avantageusement, ce mode de réalisation permet de mesurer un paramètre tel que l'atténuation des signaux ultrasonores. Ce paramètre est particulièrement significatif dans le cas du foie humain car il est lié à la présence de stéatose.
La structure et les caractéristiques de la pluralité de tirs ultrasonores sont représentées sur les figures 2 et 3.
La figure 2 montre que la pluralité de tirs est formée par K groupes de tirs ultrasonores. Par exemple dans le cas montré en figure 2, on a K=3 groupes de tirs ultrasonores.
Chacun des K groupes est formé par un nombre de blocs de tirs ultrasonores variable. On indique le nombre de blocs de tirs formant le groupe K avec la lettre $M_K$. Les trois groupes représentés sur la figure 2 sont formés respectivement par Mk=1, Mk=2 et Mk=3 blocs de tirs respectivement.
La figure 2 montre également que les K groupes de tirs ultrasonores sont séparés temporellement et ils peuvent être émis avec une cadence variable, l'écart de temps entre la fin d'un groupe et le début du suivant n'étant pas fixé.
Un avantage de ce mode de réalisation est d'assurer une grande variété de séquences possibles de tirs ultrasonores.
Chacun des K groupes de tirs ultrasonores est formé par la répétition avec une cadence PRF2 de $M_K$ bloc(s) de tirs ultrasonores, $M_K$ étant supérieur ou égale à 1.
La figure 3 montre la structure d'un des K groupes formé, à titre d'exemple, par $M_K$ = 5 blocs de tirs ultrasonores.

Comme il est visible sur la figure 3, la distance temporelle entre un bloc et le bloc successif est égale à 1/PRF2. PRF2 est donc le taux de répétition avec lequel les blocs sont émis.

Chacun desdits $M_K$ blocs est composé par N tirs ultrasonores, N étant supérieur ou égale à 1, PRF1 étant la cadence d'émission des N tirs quand N est choisi supérieur à 1. Comme il est visible sur la figure 3, les N=3 tirs ultrasonores sont émis avec une cadence fixe et égale à 1/PRF1. De plus, les N tirs ultrasonores formant un des $M_K$ blocs sont repartis sur P fréquences, P étant compris entre 1 et N. Dans le cas illustré à la figure 3 les N=3 tirs ultrasonores sont repartis sur P=3 fréquences différentes.

La figure 3 illustre qu'au moins deux tirs ultrasonores appartenant à deux blocs différents ont deux fréquences différentes. Par exemple, le premier tir du premier bloc et le deuxième tir du deuxième bloc ont deux fréquences différentes.

L'avantage de ce mode de réalisation est d'émettre des impulsions ultrasonores reparties sur plusieurs fréquences. Cela permet de mesurer P valeurs du paramètre d'atténuation ultrasonore a(f), une pour chacune des P valeurs de fréquence, et d'obtenir une mesure d'atténuation ultrasonore plus précise et reproductible. La fonction a(f) est le coefficient d'absorption du tissu en fonction de la fréquence ultrasonore f, exprimé en dB $m^{-1}$. Dans le cas du foie humain, un avantage de ce mode de réalisation est de permettre l'évaluation de la stéatose de façon plus précise et reproductible.

Selon un mode de réalisation de l'invention la cadence PRF2 est supérieure ou égale à 1Hz et la cadence PRF1 est supérieure ou égale à 1kHz.

Un avantage de ce mode de réalisation est d'utiliser une cadence PRF1 élevée, ce qui permet de sonder le tissu avec plusieurs tirs ultrasonores avant que le milieu se déplace par exemple à cause de la respiration du patient. D'ailleurs une cadence PRF2 élevée permet également d'obtenir des mesures avec un bon rapport signal sur bruit tout en utilisant de temps d'acquisition réduits, ce qui est un avantage pour des applications en milieu médical.

Selon un mode de réalisation de l'invention les P fréquences sont différentes et les largeurs spectrales des N tirs ultrasonores sont choisies pour couvrir sensiblement la bande passante du transducteur ultrasonore US.

Un avantage de ce mode de réalisation est de repartir les impulsions sur un spectre adapté pour couvrir l'intégralité de la bande passante du transducteur et donc de maximiser le rapport signal sur bruit tout en réduisant la durée de l'acquisition.

Selon un mode de réalisation particulier de l'invention, P est égal à 1. Dans ce cas les N tirs appartenant au même bloc ont la même fréquence mais les tirs appartenant à blocs différents ont des fréquences différentes.

Un avantage de ce mode de réalisation est de regrouper les tirs ultrasonores ayant la même fréquence dans le même bloc.

Selon un autre mode de réalisation de l'invention P est égale à N.

Dans ce mode de réalisation chacune des N impulsions formant un bloc a une fréquence centrale différente, ce qui est avantageux pour exploiter l'intégralité de la bande passante du transducteur et maximiser le rapport signal sur bruit.

Selon un mode de réalisation de l'invention, M est compris entre 10 et 10000, P est compris entre 2 et 5, N est compris entre 2 et 5, PRF2 est compris entre 5Hz et 500Hz et PRF1 est compris entre 3kHz et 10kHz.

Selon un mode de réalisation, Fc est la fréquence centrale du transducteur ultrasonore, P est égal à 5 et les fréquences choisies sont 0,5*Fc < F1 < 0,7*Fc, 0,7*Fc < F2 <0,9*Fc, 0,9*Fc < F3 < 1,1*Fc, 1,1*Fc < F4 <1,3*Fc, 1,3*Fc < F5 < 1,5*Fc.

Un avantage de ce mode de réalisation est de choisir des gammes de valeurs pour les fréquences centrales des impulsions ultrasonores, les gammes étant adaptées pour couvrir la bande passante du transducteur. Cela permet de maximiser le rapport signal sur bruit pour une bande passante du transducteur donnée.

Par exemple si Fc = 3.5 MHz, les gammes de valeurs selon ce mode de réalisation couvrent avantageusement la bande passante du transducteur formant la sonde M du Fibroscan™.

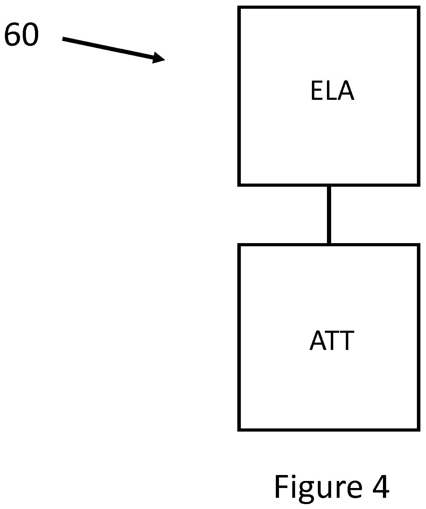

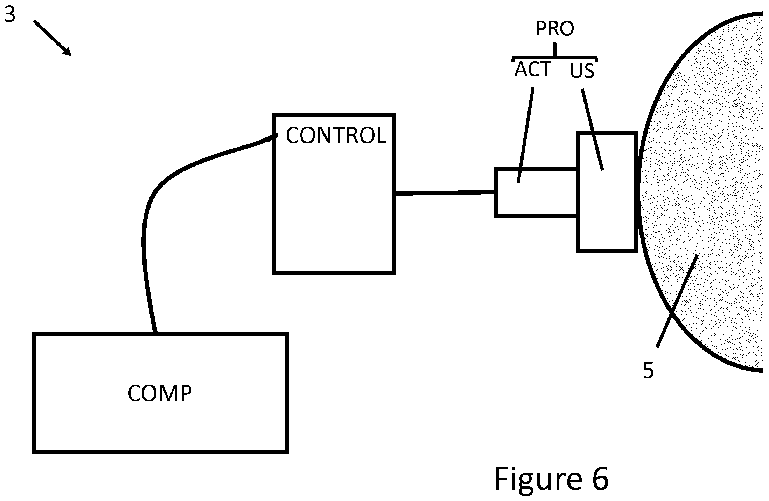

La figure 4 illustre les différentes étapes du deuxième procédé selon l'invention 60 de mesure d'au moins un paramètre viscoélastique d'un organe humain ou animal (cf. la référence 5 de la figure 6 décrite ultérieurement), ledit procédé comportant les étapes suivantes :

- mesure ELA d'un paramètre viscoélastique du foie par élastographie impulsionnelle ;

- mesure ATT d'un paramètre d'atténuation des ultrasons avec le procédé 1 de la figure 1 ;

On entend par élastographie impulsionnelle une technique de type « Vibration Controlled Transient Elastography » ou VCTE. Avec cette technologie, implémentée dans le Fibroscan™, il est possible de mesurer de façon non invasive l'élasticité d'un tissu biologique comme il est décrit par exemple dans les brevets EP 1 169 636 et EP 1 531 733.

L'étape ELA consiste donc à générer mécaniquement une onde de cisaillement qui se propage à l'intérieur du tissu à caractériser. La propagation de l'onde de cisaillement est ensuite suivie en émettant dans le tissu des impulsions ultrasonores. Grâce aux ultrasons réfléchis par le milieu, les déplacements induits dans le tissu par l'onde de cisaillement sont mesurés. En envoyant des séquences d'ultrasons on peut mesurer la vitesse de propagation de l'onde de cisaillement qui est liée à l'élasticité du milieu.

L'étape ATT consiste à mettre en œuvre le procédé 1 décrit par la présente demande et illustré par la figure 1.

Le procédé 60 selon ce mode de réalisation est particulièrement avantageux car il permet de mesurer deux paramètres viscoélastiques, à savoir l'élasticité et l'atténuation ultrasonore, avec une seule réalisation du procédé 60.

Un autre avantage du procédé 60 dans le cas du foie humain est d'associer systématiquement une mesure d'élasticité du tissu à une évaluation de l'atténuation ultrasonore, ce qui peut être particulièrement utile pour évaluer la progression de la fibrose et de la stéatose.

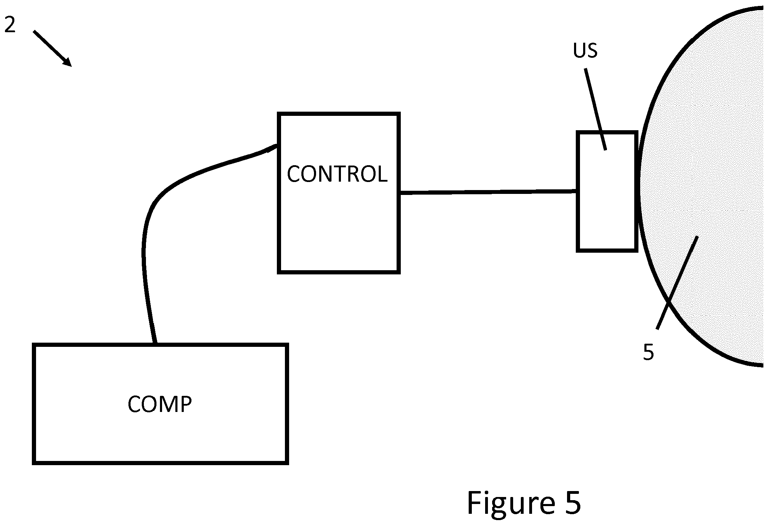

La figure 5 illustre schématiquement un exemple de dispositif 2 pour la mise en œuvre du procédé 1 selon l'invention de la figure 1. Ce dispositif comporte :

- un transducteur US ultrasonore adapté à l'émission et à la réception en temps réel de signaux ultrasonores et adapté pour être disposé au contact d'une surface extérieure d'un milieu viscoélastique 5 ;

- des moyens de commande CONTROL dudit transducteur ultrasonore US pour l'émission d'une pluralité de tirs ultrasonores, ladite pluralité de tirs ultrasonores étant formée par K groupes de tirs, lesdits groupes étant séparés temporellement, K étant supérieur ou égal à 1, chacun des K groupes de tirs ultrasonores étant formé par la répétition avec une cadence PRF2 de $M_K$ groupe(s) de tirs ultrasonores, $M_K$ étant supérieur ou égale à 1 ; chacun desdits $M_K$ blocs étant composé par N tirs ultrasonores, N étant supérieur ou égale à 1, PRF1 étant la cadence d'émission des N tirs quand N est choisi supérieur à 1 ; les N tirs ultrasonores étant répartis sur P fréquences différentes, P étant compris entre 1 et N;

- des moyens d'enregistrement et de traitement COMP des signaux ultrasonores réfléchis adaptés pour déterminer au moins un paramètre viscoélastique de l'organe 5.

Le transducteur US est utilisé pour l'émission des impulsions ultrasonores se propageant dans le foie. Ce transducteur est adapté pour l'émission d'impulsions ultrasonores ayant plusieurs fréquences centrales et il est disposé à proximité du tissu 5. Le transducteur US a également le rôle de capteur pour les signaux ultrasonores réfléchis.

Les moyens de commande CONTROL ont le rôle de piloter le transducteur US pour l'émission d'une séquence d'impulsions ultrasonores multifréquence. Les paramètres de la séquence, à savoir PRF1, PRF2, $M_K$, N, P et les valeurs des fréquences centrales des impulsions sont choisis par l'opérateur et mis en œuvre grâce aux moyens CONTROL.

Les moyens d'enregistrement et de traitement COMP des signaux ultrasonores réfléchis permettent de calculer, à partir des données enregistrées, les paramètres d'atténuation. Le coefficient d'atténuation ultrasonore, ou paramètre a(f), peut par exemple être calculée grâce à l'algorithme d'obtention du paramètre d'atténuation contrôlé (ou Controlled Attenuation Parameter ou CAP).

Par exemple les moyens CONTROL et COMP peuvent être intégrés dans un ordinateur ou n'importe quel dispositif programmé qui permettrait à la fois la programmation des paramètres de la séquence d'impulsions ultrasonores, l'actionnement et le contrôle du dispositif 2 et l'enregistrement et le traitement des données.

Avantageusement ce dispositif permet l'émission d'impulsions ultrasonores multifréquences et la mesure de leur atténuation selon le procédé 1. Le dispositif 2 permet donc de mesurer a(f) pour plusieurs valeurs de la fréquence centrale f des impulsions. Dans le cas du foie humain, cela permet une évaluation plus précise et fiable de la stéatose du foie, c'est-à-dire de la concentration de lipides dans le tissu.

La figure 6 illustre schématiquement un exemple de dispositif 3 pour la mise en œuvre du procédé 60 illustré par la figure 4. Le dispositif 3 comporte :

- un vibreur ACT adapté pour appliquer à un organe 5 humain ou animal une impulsion basse fréquence pour la génération d'une onde de cisaillement ;

- une sonde PRO comprenant au moins un transducteur 10 ultrasonore adapté à l'émission et à la réception en temps réel de signaux ultrasonores et adapté pour être disposé au contact d'une surface extérieure du milieu viscoélastique 5 ;

- des moyens de commande CONTROL de ladite sonde PRO pour l'émission d'une pluralité de tirs ultrasonores, ladite pluralité de tirs ultrasonores étant formée par K groupes de tirs, lesdits groupes étant séparés temporellement, K étant supérieur ou égal à 1, chacun des K groupes de tirs ultrasonores étant formé par la répétition avec une cadence PRF2 de $M_K$ groupe(s) de tirs ultrasonores, $M_K$ étant supérieur ou égale à 1 ; chacun desdits $M_K$ blocs étant composé par N tirs ultrasonores, N étant supérieur ou égale à 1, PRF1 étant la cadence d'émission des N tirs quand N est choisi supérieur à 1 ; les N tirs ultrasonores étant répartis sur P fréquences différentes, P étant compris entre 1 et N;

- des moyens d'enregistrement et de traitement COMP des signaux ultrasonores réfléchis adaptés pour déterminer au moins un paramètre viscoélastique de l'organe 5.

Le dispositif 3 permet la mise en œuvre du procédé 60 selon l'invention et donc la mesure de deux paramètres viscoélastiques du tissu. Par exemple, le premier paramètre viscoélastique peut être un paramètre décrivant la propagation d'une onde de cisaillement dans le tissu et le deuxième un paramètre d'atténuation des ultrasons.

On entend par vibreur ACT un actuateur mécanique capable de générer une onde de cisaillement se propageant dans le tissu. Cette onde de cisaillement est générée en appliquant un déplacement impulsionnel au transducteur US qui se trouve à proximité du tissu 5.

Le transducteur US émet des ultrasons se propageant dans le tissu 5. Ces ultrasons peuvent être monofréquence pour suivre la propagation de l'onde de cisaillement. Alternativement des impulsions multifréquence peuvent être utilisées pour la mesure du paramètre d'atténuation ultrasonore.

Selon le procédé 60 de mise en œuvre de l'invention le premier paramètre viscoélastique est déterminé par élastographie impulsionnelle au cours de l'étape ELA, selon la technologie dite Vibration Controlled Transient Elastographie (VCTE). Cette étape est réalisée à l'aide du dispositif 3. Le vibreur ACT génère une onde de cisaillement en appliquant un déplacement impulsionnel du transducteur US, ladite onde de cisaillement se propageant dans le tissu à caractériser. Les ultrasons monofréquence générés par le transducteur US sont utilisés pour suivre la propagation de l'onde de cisaillement. Cette méthode permet de déduire des paramètres comme la vitesse de propagation de l'onde de cisaillement. Par exemple, dans le cas du foie humain cette méthode permet de mesurer l'élasticité de l'organe et donc d'avoir des informations sur la fibrose.

Le dispositif 3 permet également de réaliser l'étape ATT du procédé 60 selon l'invention. Au cours de cette étape le procédé 1 est mis en œuvre, ce qui revient à envoyer une séquence d'impulsions ultrasonores multifréquence et mesurer les signaux réfléchis pour déterminer les paramètres d'atténuation aux différentes fréquences. Au cours de l'étape ATT du procédé 60 seul le transducteur US est actif.

Avantageusement le dispositif 3 permet de mettre en œuvre le procédé 60 et d'intercaler des mesures d'élastographie impulsionnelle et des mesures d'atténuation ultrasonore. La possibilité de réaliser ces deux mesures en série comme décrit par le procédé 60 et avec le même dispositif, permet de réduire la durée des examens tout en réduisant leur impact sur la vie des patients.

De plus, dans le cas du foie humain l'évaluation simultanée de la stéatose et de la fibrose permet d'établir un diagnostic complet.

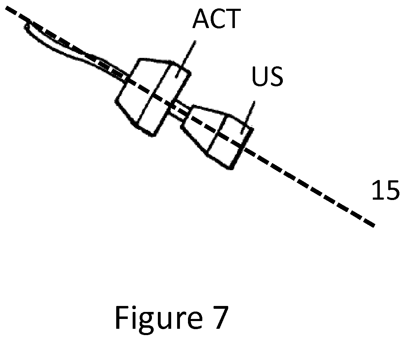

La figure 7 montre un exemple de sonde PRO du dispositif 3 dans lequel le vibreur ACT et le transducteur US sont solidarisés. De plus, le vibreur ACT et le transducteur US ont un axe de symétrie 15 commun, ce qui garantit que la direction de propagation de l'onde de cisaillement est parallèle à la direction de propagation des ultrasons. Cette condition de parallélisme est nécessaire pour mesurer des valeurs correctes de la vitesse de propagation de l'onde de cisaillement.

**Revendications**

1. Procédé (1) de mesure d'au moins un paramètre viscoélastique d'un organe (5) humain ou animal, ledit procédé comportant les étapes suivantes :

   • émission (EM-US) par un transducteur ultrasonore (US) d'une pluralité de tirs ultrasonores, lesdits tirs ultrasonores se propageant dans l'organe à caractériser ;
   • réception (REC) par le transducteur ultrasonore (US) et enregistrement des signaux ultrasonores réfléchis ;
   • détermination (DET) d'au moins un paramètre viscoélastique de l'organe (5) à partir des signaux ultrasonores enregistrés ;

   le procédé (1) étant **caractérisé en ce que** :

   • la pluralité de tirs ultrasonores est formée par K groupes de tirs, lesdits groupes étant séparés temporellement, K étant supérieur ou égal à 1 ;
   • chacun des K groupes de tirs ultrasonores est formé par la répétition avec une cadence PRF2 de $M_K$ bloc(s) de tirs ultrasonores, $M_K$ étant supérieur ou égale à 1 ;
   • chacun desdits $M_K$ blocs est composé par N tirs ultrasonores, N étant supérieur ou égale à 1, PRF1 étant la cadence d'émission des N tirs quand N est choisi supérieur à 1 ;
   • les N tirs ultrasonores sont repartis sur P fréquences, P étant compris entre 1 et N, au moins deux tirs ultrasonores appartenant à deux blocs différents ayant fréquences différentes.

2. Procédé (1) selon la revendication 1 **caractérisé en ce que** le paramètre viscoélastique mesuré est un paramètre d'atténuation ultrasonore.

3. Procédé (1) selon l'une des revendications précédentes **caractérisé en ce que** la cadence PRF2 est supérieure ou égale à 1Hz et la cadence PRF1 est supérieure ou égale à 1kHz.

4. Procédé (1) selon l'une des revendications précédentes **caractérisé en ce que** les P fréquences différentes et les largeurs spectrales des N tirs ultrasonores sont choisies pour couvrir sensiblement la bande passante du transducteur ultrasonore (US).

5. Procédé (1) selon la revendication 4 **caractérisé en ce que** P est égal à N.

6. Procédé (1) selon l'une des revendications 1 à 4 **caractérisé en ce que** M est compris entre 10 et 10000, P est compris entre 2 et 11, N est compris entre 2 et 11, PRF2 est compris entre 5Hz et 500Hz et PRF1 est compris entre 3kHz et 10kHz.

**7.** Procédé (1) selon l'une des revendications précédentes **caractérisé en ce que** Fc est la fréquence centrale du transducteur ultrasonore, P est égal à 5 et les fréquences choisies sont 0,5*Fc < F1 < 0,7*Fc, 0,7*Fc < F2 <0,9*Fc, 0,9*Fc < F3 < 1,1*Fc, 1,1*Fc < F4 <1,3*Fc, 1,3*Fc < F5 < 1,5*Fc.

**8.** Procédé (60) de mesure d'au moins un paramètre viscoélastique d'un organe humain ou animal (5), ledit procédé comportant les étapes suivantes :

> • mesure (ELA) d'un paramètre viscoélastique de l'organe (5) par mise en oeuvre d'une technique d'élastographie impulsionnelle ;
> • mesure (ATT) d'un paramètre d'atténuation des ultrasons avec le procédé (1) selon la revendication 2 ou selon l'une des revendications 3 à 7 prise dans la dépendance de la revendication 2.

**9.** Dispositif (2) pour la mise en œuvre du procédé (1) selon les revendications 1 à 7, ledit dispositif comportant :

> a. un transducteur (US) ultrasonore adapté à l'émission et à la réception en temps réel de signaux ultrasonores et adapté pour être disposé au contact d'une surface extérieure du milieu viscoélastique (5) ;
> b. des moyens de commande (CONTROL) dudit transducteur ultrasonore (US) pour l'émission d'une pluralité de tirs ultrasonores, ladite pluralité de tirs ultrasonores étant formée par K groupes de tirs, lesdits groupes étant séparés temporellement, K étant supérieur ou égal à 1, chacun des K groupes de tirs ultrasonores étant formé par la répétition avec une cadence PRF2 de $M_K$ groupe(s) de tirs ultrasonores, $M_K$ étant supérieur ou égale à 1 ; chacun desdits $M_K$ blocs étant composé par N tirs ultrasonores, N étant supérieur ou égale à 1, PRF1 étant la cadence d'émission des N tirs quand N est choisi supérieur à 1 ; les N tirs ultrasonores étant répartis sur P fréquences, P étant compris entre 1 et N, au moins deux tirs ultrasonores appartenant à deux blocs différents ayant fréquences différentes ;
> c. des moyens d'enregistrement et de traitement (COMP) des signaux ultrasonores réfléchis adaptés pour déterminer au moins un paramètre viscoélastique de l'organe (5).

**10.** Dispositif (3) selon la revendication 9 pour la mise en œuvre du procédé (60) selon la revendication 8, ledit dispositif comportant en outre :
un vibreur (ACT) adapté pour appliquer à un organe (5) humain ou animal une impulsion basse fréquence pour la génération d'une onde de cisaillement.

**Patentansprüche**

**1.** Messverfahren (1) wenigstens einen viskoelastischen Parameters eines menschlichen oder tierischen Organs (5), wobei das genannte Verfahren die folgenden Schritte umfasst:

> • Ausgabe (EM-US) einer Vielzahl von Ultraschallschüssen durch einen Ultraschall-Messumformer (US), wobei die genannten Ultraschallschüsse sich in einem zu kennzeichnenden Organ verbreiten;
> • Empfang (REC) durch den Ultraschall-Messumformer (US) und Abspeicherung der zurückgeworfenen Ultraschallsignale;
> • Bestimmung (DET) wenigstens eines viskoelastischen Parameters des Organs (5) ausgehend von den abgespeicherten Ultraschallsignalen;

wobei das Verfahren (1) **dadurch gekennzeichnet ist, dass**:

> • die Vielzahl von Ultraschallschüssen durch K Schussgruppen gebildet wird, wobei die Gruppen vorübergehend getrennt werden, wobei K größer als oder gleich 1 ist;
> • jede der K Ultraschall-Schussgruppen durch die Wiederholung mit einer Kadenz PRF2 von $M_k$ Ultraschall-Schussblock (-blöcken) gebildet wird, wobei $M_K$ größer als oder gleich 1 ist;
> • jeder der genannten $M_K$ Blöcke aus N Ultraschall-Schüssen zusammengesetzt wird, wobei N größer als oder gleich 1 ist, wobei PRF1 die Sendekadenz der N Schüsse ist, wenn N größer als 1 gewählt wird;
> • wobei die N Ultraschallschüsse über P Frequenzen verteilt werden, wobei P zwischen 1 und N inbegriffen ist, wobei wenigstens zwei Ultraschallschüsse zu zwei unterschiedlichen Blöcken gehören, die unterschiedliche Frequenzen aufweisen.

**2.** Verfahren (1) gemäß Anspruch 1, **dadurch gekennzeichnet, dass** der gemessene viskoelastische Parameter ein Ultraschall-Abschwächungsparameter ist.

**3.** Verfahren (1) gemäß einem der voranstehenden Ansprüche, **dadurch gekennzeichnet, dass** die Kadenz PRF2 größer als oder gleich 1 Hz ist und dass die Kadenz PRF1 größer als oder gleich 1 kHz ist.

**4.** Verfahren (1) gemäß einem der voranstehenden Ansprüche, **dadurch gekennzeichnet, dass** die P unterschiedlichen Frequenzen und die spektralen Breiten der N Ultraschallschüsse ausgewählt sind, um die Bandbreite des Ultraschall-Messumformers

**5.** Verfahren (1) gemäß Anspruch 4, **dadurch gekennzeichnet, dass** P gleich N ist.

**6.** Verfahren (1) gemäß einem der Ansprüche 1 bis 4, **dadurch gekennzeichnet, dass** M zwischen 10 und 10.000 inbegriffen ist, P zwischen 2 und 11 inbegriffen ist, N zwischen 2 und 11 inbegriffen ist, PRF2 zwischen 5 Hz und 500 Hz inbegriffen ist und PRF1 zwischen 3 kHz und 10 kHz inbegriffen ist.

**7.** Verfahren (1) gemäß einem der voranstehenden Ansprüche, **dadurch gekennzeichnet, dass** Fc die zentrale Frequenz des Ultraschall-Messumformers ist, P gleich 5 ist und die ausgewählten Frequenzen $0,5 \times Fc < F1 < 0,7 \times Fc$, $0,7 \times Fc < F2 < 0,9 \times Fc$, $0,9 \times Fc < F3 < 1,1 \times Fc$, $1,1 \times Fc < F4 < 1,3 \times Fc$, $1,3 \times Fc < F5 < 1,5 \times Fc$ sind.

**8.** Messverfahren (60) wenigstens eines viskoelastischen Parameters eines menschlichen oder tierischen Organs (6), wobei das genannte Verfahren die folgenden Schritte umfasst:

• Messung (ELA) eines viskoelastischen Parameters des Organs (5) per Umsetzen einer Impulselastografie-Technik;
• Messung (ATT) eines Abschwächungsparameters des Ultraschalls mit dem Verfahren (1) gemäß Anspruch 2 oder gemäß einem der Ansprüche 3 bis 7 in Abhängigkeit von dem Anspruch 2.

**9.** Verfahren (2) für die Umsetzung des Verfahrens (1) gemäß Anspruch 1 bis 7, wobei die genannte Vorrichtung umfasst:

a. einen Ultraschall-Messumformer (US), der zum Senden und zum Empfangen von Ultraschallsignalen in Echtzeit geeignet ist und geeignet ist, um in Kontakt mit einer Außenfläche des viskoelastischen Materials (5) angeordnet zu sein;
b. Steuermittel (CONTROL) des genannten Ultraschall-Messumformers (US) zum Senden einer Vielzahl von Ultraschallschüssen, wobei die genannte Vielzahl von Ultraschallschüssen durch K Schussgruppen gebildet wird, wobei die genannten Gruppen vorübergehend getrennt werden, wobei K größer als oder gleich 1 ist, wobei jede der K Ultraschallschussgruppen durch die Wiederholung mit einer Kadenz PRF2 von $M_K$ Ultraschallgruppe (-gruppen) gebildet wird, wobei $M_K$ größer als oder gleich 1 ist; wobei jeder der genannten $M_K$ Blöcke aus N Ultraschallschüssen zusammengesetzt ist, wobei N größer als oder gleich 1 ist, wobei PRF1 die Sendekadenz der N Schüsse ist, wenn N größer als 1 ausgewählt wird; wobei die N Ultraschallschüsse über P Frequenzen verteilt werden, wobei P zwischen 1 und N inbegriffen ist, wobei wenigstens zwei Ultraschallschüsse zu zwei unterschiedlichen Blöcken mit unterschiedlichen Frequenzen gehören;
c. Speicher- und Verarbeitungsmittel (COMP) der zurückgeworfenen Ultraschallsignale, die geeignet sind, um wenigstens einen viskoelastischen Parameter des Organs (5) zu bestimmen.

**10.** Vorrichtung (3) gemäß Anspruch 9 für die Umsetzung des Verfahrens (60) gemäß Anspruch 8, wobei die genannte Vorrichtung darüber hinaus umfasst: einen Vibrator (ACT), der zum Anwenden eines Niedrigfrequenz-Impulses auf ein menschliches oder tierisches Organ (5) zum Erzeugen einer Abscherwelle geeignet ist.

**Claims**

**1.** Method (1) for measuring at least one viscoelastic parameter of a human or animal organ (5), wherein said method includes the following steps:

• emission (EM-US) by an ultrasound transducer (US) of a plurality of ultrasound shots, wherein said ultrasound shots are propagated in the organ to be characterised;
• reception (REC) by the ultrasound transducer (US) and recording of the reflected ultrasound signals;
• determination (DET) of at least one viscoelastic parameter of the organ (5) from the recorded ultrasound signals;

the method (1) being **characterised in that**:

• the plurality of ultrasound shots is formed by K groups of shots, wherein said groups are separated over time, and wherein K is greater than or equal to 1;
• each of the K groups of ultrasound shots is formed by the repetition at a rate PRF2 of $M_K$ set(s) of ultrasound shots, wherein $M_K$ is greater than or equal to 1;
• each of the said $M_K$ sets is composed of N ultrasound shots, wherein N is greater than or equal to 1, and wherein PRF1 is the rate of emission of the N shots when N is chosen to be greater than 1;
• the N ultrasound shots are distributed over P frequencies, wherein P is between 1 and N, and wherein at least two ultrasound shots belonging to two different sets have different frequencies.

**2.** Method (1) according to claim 1, **characterised in that** the measured viscoelastic parameter is an ultrasound attenuation parameter.

**3.** Method (1) according to one of the previous claims, **characterised in that** rate PRF2 is greater than or equal to 1 Hz and **in that** rate PRF1 is greater than or equal to 1 kHz.

**4.** Method (1) according to one of the previous claims, **characterised in that** the P different frequencies, and the spectral widths of the N ultrasound shots, are chosen such that they roughly cover the bandwidth of the ultrasound transducer (US).

**5.** Method (1) according to claim 4 **characterised in that** P is equal to N.

**6.** Method (1) according to one of claims 1 to 4 **characterised in that** M is between 10 and 10000, P is between 2 and 11, N is between 2 and 11, PRF2 is between 5 Hz and 500 Hz and PRF1 is between 3 kHz and 10 kHz.

**7.** Method (1) according to one of the previous claims, **characterised in that** Fc is the central frequency of the ultrasound transducer, P is equal to 5, and the chosen frequencies are $0.5{*}Fc < F1 < 0.7{*}Fc$, $0.7{*}Fc < F2 < 0.9{*}Fc$, $0.9{*}Fc < F3 < 1.1{*}Fc$, $1.1{*}Fc < F4 < 1.3{*}Fc$, $1.3{*}Fc < F5 < 1.5{*}Fc$.

**8.** Method (60) for measuring at least one viscoelastic parameter of a human or animal organ (5), wherein said method includes the following steps:

　• measurement (ELA) of a viscoelastic parameter of the organ (5) by transient elastography;
　• measurement (ATT) of an ultrasound attenuation parameter according to the method of claim 2 or to the method of any of claims 3 to 7 considered in the dependency of claim 2.

**9.** Device (2) for implementation of the method (1) according to claims 1 to 7, wherein said device includes:

　a. an ultrasound transducer (US) able to emit and receive ultrasound signals, in real time, and suitable to be positioned in contact with an outside surface of the viscoelastic medium (5);
　b. means of control (CONTROL) of said ultrasound transducer (US) for the emission of a plurality of ultrasound shots, wherein said plurality of ultrasound shots is formed by K groups of shots, wherein said groups are separated over time, wherein K is greater than or equal to 1, wherein each of the K groups of ultrasound shots is formed by the repetition at a rate PRF2 of $M_K$ group(s) of ultrasound shots, wherein $M_K$ is greater than or equal to 1; wherein each of the said $M_K$ sets is composed of N ultrasound shots, wherein N is greater than or equal to 1, and wherein PRF1 is the rate of emission of the N shots when N is chosen to be greater than 1; wherein the N ultrasound shots are distributed over P frequencies, wherein P is between 1 and N, and wherein at least two ultrasound shots belonging to two different sets have different frequencies;
　c. means (COMP) of recording and processing the reflected ultrasound signals, able to determine at least one viscoelastic parameter of organ (5).

**10.** Device (3) according to claim 9, for implementation of the method (60) according to claim 8, wherein said device further includes: a vibrator (ACT) able to apply to a human or animal organ (5) a low-frequency pulse to generate a shear wave.

EP 3 487 412 B1

1

```
┌─────────────────┐
│                 │
│     EM-US       │
│                 │
└────────┬────────┘
         │
┌────────┴────────┐
│                 │
│      REC        │
│                 │
└────────┬────────┘
         │
┌────────┴────────┐
│                 │
│      DET        │
│                 │
└─────────────────┘
```

Figure 1

Figure 2

temps

1/PRF1

1/PRF2

N=3 N=3 N=3 N=3 N=3

$M_K = 5$

Figure 3

EP 3 487 412 B1

60 →

ELA

ATT

Figure 4

Figure 5

EP 3 487 412 B1

3

PRO

ACT    US

CONTROL

COMP

5

Figure 6

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1169636 A
- EP 1531733 A
- EP 2477551 A
- US 20150374338 A

**Littérature non-brevet citée dans la description**

- **M. SASSO et al.** Controlled attenuation parameter (cap): a novel vcte guided ultrasonic attenuation measurement for the evaluation of hepatic steatosis: preliminary study and validation in a cohort of patients with chronic liver disease from various causes. *Ultrasound in Medicicine and Biology,* 2010, vol. 36 (11
- **M. SASSO et al.** Liver steatosis assessed by controlled attenuation parameter (cap) measured with the xl probe of the fibroscan: a pilot study assessing diagnostic accuracy. *Ultrasound in Medicine and Biology,* 2015, vol. 42 (1